# EUROPEAN PATENT APPLICATION

(11) **EP 3 673 901 A1**
(43) Date of publication of application: **01.07.2020**
(21) Application number: 18848252.5
(22) Date of filing: 20.08.2018
(51) Int. Cl.: A61K 9/20

(54) **PHARMACEUTICAL COMPOSITION PARTICLES, ORALLY DISINTEGRATING PREPARATION CONTAINING SAME, AND METHOD FOR PRODUCING PHARMACEUTICAL COMPOSITION PARTICLES**

(30) Priority: 21.08.2017 JP 2017158339
(71) Applicant: Nipro Corporation, Osaka-shi, Osaka 531-8510 (JP); Zensei Pharmaceutical Industries Co., Ltd., Sakai-shi, Osaka 593-8307 (JP)
(72) Inventor: HAYASHIDA, Tomohiro, Osaka-shi Osaka 531-8510 (JP); HOASHI, Yohei, Osaka-shi Osaka 531-8510 (JP); IJITSU, Shin, Osaka-shi Osaka 531-8510 (JP); NAKANO, Yoshio, Kishiwada-shi Osaka 596-0808 (JP); YAMAZAKI, Junji, Kishiwada-shi Osaka 596-0808 (JP); INOUE, Katsuhisa, Kishiwada-shi Osaka 596-0808 (JP); AIZAWA, Atsushi, Kishiwada-shi Osaka 596-0808 (JP)
(74) Representative: Reitstötter Kinzebach
(86) International application number: PCT/JP2018/030618
(87) International publication number: WO 2019/039420

(57) **Abstract**

Provided are a pharmaceutical composition particle which is capable of achieving both masking of an unpalatable taste and improvement in dissolution properties, an orally disintegrating tablet containing the pharmaceutical composition particle, and a method for manufacturing a pharmaceutical composition particle. The pharmaceutical composition particle includes a core particle containing a drug and a water-soluble gelling swelling substance; and an outer layer which contains a water-insoluble substance and constitutes a coating provided on the outer side of the core particle. The method for manufacturing a pharmaceutical composition particle according to the present invention includes: a pulverization step of pulverizing a water-soluble gelling swelling substance so that the pulverized water-soluble gelling swelling substance has an average particle diameter of 15 µm or less; a core particle manufacturing step of mixing the water-soluble gelling swelling substance pulverized in the pulverization step and a drug to manufacture a core particle; and an outer layer formation step of coating the core particle with a water-insoluble substance on the outer side of the core particle to form an outer layer.

## Description

### Technical Field

The present invention relates generally to a pharmaceutical composition particle for oral administration, an orally disintegrating tablet containing the pharmaceutical composition particle, and a method for manufacturing the pharmaceutical composition particle. In particular, the present invention relates to a pharmaceutical composition particle for oral administration, which is release-controllable for masking of an unpalatable taste and improvement in dissolution properties; an orally disintegrating tablet containing the pharmaceutical composition particle; and a method for manufacturing the pharmaceutical composition particle.

### Background Art

Dosage forms of oral pharmaceutical composition particles such as granules, fine granules, and powder have sizes smaller than those of a tablet and a capsule. These dosage forms allow even patients who have difficulty in swallowing of the tablet and the capsule to take the oral pharmaceutical composition particles easily. In recent years, dosage forms such as the above-mentioned dosage forms which offer an improved convenience for patients and hospitals have been attracting attention. Among the highly convenient dosage forms, an orally disintegrating tablet is highly convenient in that the orally disintegrating tablet is capable of being administered without water, easy to swallow, and suitable for tube administration. Therefore, a variety of improvements have been made on a method for manufacturing fine particles which form the orally disintegrating tablet.

Pharmaceutical composition particles, in particular, fine particles having an average particle diameter of 400 µm or less have a large surface area per weight as compared with fine particles having an average particle diameter which is larger than that of the above-mentioned particles. In other words, the fine particles having a small size have a large area that is in contact with water in the oral cavity as compared with fine particles having a relatively large size, and the smaller fine particles are more rapidly infiltrated with water. Therefore, when the pharmaceutical composition particle having a small size is administered, the drug tends to be released rapidly in the oral cavity. This may cause a variety of problems. For example, when a drug has an unpalatable taste, the drug which has been rapidly released in the oral cavity may cause a patient to feel a great deal of discomfort, which may result in considerable decrease in administration compliance. In addition, when a drug to be absorbed in the oral cavity is included in the pharmaceutical composition particles, the drug has been rapidly released in the oral cavity may cause problems such as side effects and variation in drug efficacy among individuals.

In order to avoid these problems, it is required to delay drug release of the pharmaceutical composition particles in the oral cavity for a predetermined time. By suppressing the drug release for the predetermined time during which the pharmaceutical composition particles stay in the oral cavity, masking of the unpalatable taste becomes possible. In addition, the problems such as the side effects and the variation in drug efficacy among individuals can also be avoided.

On the other hand, in order to enable a drug to show its drug efficacy sufficiently, it is necessary that the drug is released from oral pharmaceutical composition particles, and a sufficient quantity of the drug is absorbed into the body. An orally administered preparation moves through the gastrointestinal tract over time. In general, most drugs are absorbed in the upper part of the gastrointestinal tract.

When these are considered, it is desired that after suppressing the drug release from the pharmaceutical composition particles for the predetermined time, the pharmaceutical composition particles are disintegrated to release the drug as rapidly as possible, and the drug is absorbed in the upper part of the gastrointestinal tract.

Accordingly, in order to achieve the purposes such as masking of the unpalatable taste of a drug and avoiding of absorption of the drug in the oral cavity, it is required to suppress the drug release in the oral cavity for a predetermined time. On the other hand, it is important that the drug is rapidly released from the oral pharmaceutical composition particles in the gastrointestinal tract. In addition, a degree of the unpalatable taste, duration of the unpalatable taste, and an absorption rate in the oral cavity vary among drugs. Thus, in designing the pharmaceutical composition particles, it is extremely important that in accordance with properties of a drug included in the pharmaceutical composition particles, an optimum combination of the time at which the drug release begins in the oral cavity and the time of drug release after oral administration is achieved.

In order to achieve the above-mentioned optimum combination, it is required to design pharmaceutical composition particles which allow a time during which the initial drug release is suppressed (hereinafter, also referred to as a "lag time") to be suitably controlled and which release the drug at a desired rate at a predetermined time after oral administration in accordance with properties of the drug and preparation. In other words, a technology for freely controlling the combinations of the time during which the drug release in the oral cavity is suppressed and the drug release rate in the body has been desired.

The orally disintegrating tablet which has attracted attention from the viewpoint of convenience is designed to contain pharmaceutical composition particles showing controlled drug release for the purpose of masking an unpalatable taste or the like. In order to reduce a rough feeling in the oral cavity, the pharmaceutical composition particles contained in the orally disintegrating tablet are required to have a size much smaller than the size of oral pharmaceutical composition particles in granules, fine granules, powder, or the like, and specifically have an average particle diameter of 400 µm or less, and preferably 300 µm or less. However, a drug contained in the pharmaceutical composition particles is tend to be released more rapidly than required as the size of the pharmaceutical composition particles becomes smaller. Therefore, in order to adequately control the combination of the time during which the drug release in the oral cavity is suppressed and the drug release rate in the body, a highly advanced drug formulation technique is required. Actually, it has been extremely difficult for a conventional formulation method to satisfy both "suppression of the initial drug release (controlling of lag time)" and "rapid drug release after the lag time" of the above-mentioned minute oral pharmaceutical composition particles.

In general, in order to mask an unpalatable taste by controlling drug release of oral pharmaceutical composition particles, a method for coating the particles with various kinds of film forming materials is used. For example, when the oral pharmaceutical composition particles containing a drug having an unpalatable taste are coated with a water-insoluble polymer, infiltration of water into each of the particles is suppressed, and thereby release of the drug in each of the particles is suppressed and the unpalatable taste is masked.

In the above-described method, even when the particle is infiltrated with water, the film formed of the water-insoluble polymer is not broken and the drug release rate remains suppressed. Therefore, the rapid drug release after the lag time cannot be achieved. On the other hand, if the amount of the film is reduced to achieve the rapid drug release, the initial drug release cannot be suppressed and the unpalatable taste cannot be masked. In other words, when drug-containing particles are simply coated with a water-insoluble polymer, it is not possible to achieve both the "suppression of the initial drug release" and the "rapid drug release after the lag time".

As a method for achieving both the suppression of the initial drug release and the rapid drug release after the lag time, for example, as described in WO 02/96392 A (Patent Literature 1), there is a method in which pharmaceutical composition particles are coated with a mixed film of a water-insoluble polymer and a water-soluble polymer. In the formulation described in Patent Literature 1, it is expected that infiltration of water into each of the particles is suppressed until the water-soluble polymer in the film is dissolved, and thereby drug release is suppressed and an unpalatable taste is masked.

For the purpose of further ensuring the drug release in the gastrointestinal tract, for example, as described in JP 2007-63263 A (Patent Literature 2), there is a method in which pharmaceutical composition particles are coated with a gastrosoluble polymer or an enteric polymer as a water-insoluble polymer.

In addition, JP 2008-214334 A (Patent Literature 3) discloses, for the purpose of improving a degree of disintegration of a film, a method in which a disintegrant and an aggregation preventing agent, together with a water-insoluble polymer, are used in combination. JP 2008-260712 A (Patent Literature 4) discloses a method in which an acidic substance or a basic substance is used together with a water-insoluble polymer in combination. JP 2011-063627 A (Patent Literature 5) discloses a method in which a saccharide is used together with a water-insoluble polymer in combination.

JP 2000-191519 A (Patent Literature 6) discloses rapid-release granules each having a core particle containing a drug in which the core particle is coated with a two-layered coating layer. Patent Literature 6 discloses that initial drug dissolution is suppressed by coating a mixed film of a water-insoluble substance and a water-soluble substance with a water-soluble substance as a second layer, in which the water-soluble substance is provided on the mixed film.

In addition, a method in which a drug-containing particle prepared by blending a water-insoluble water-swellable substance which is widely used as a disintegrant is coated with a water-insoluble film as an outer layer is proposed. For example, JP 2011-225468 A (Patent Literature 7) discloses a granular pharmaceutical composition that contains a carrier, as a core particle, which is prepared by blending a water-swellable substance which is widely used as a disintegrant; an active ingredient layer which contains a drug and is formed around the carrier; and a coating layer which contains a gastrosoluble polymer and is formed around the active ingredient layer. JP H03-130214 A (Patent Literature 8) discloses a rapid release preparation which contains a core containing a drug having an unpalatable taste and a water-swellable substance, in which the core is coated with a mixture of ethylcellulose and a water-soluble substance, in which the mixture is provided around the core. WO 2012/036078 A (Patent Literature 9) discloses a drug-containing film-coated particle having two or more layers including: (A) a film layer containing, on (P) a core particle containing no drug and no water-swellable polymer, a drug having an unpalatable taste and a water-swellable polymer and (B) a film layer containing a water-insoluble polymer, a water-soluble substance, and an inorganic compound, which is characterized in that the film layer (A) of the two or more layers is an innermost film layer. JP 2007-532623 A (Patent Literature 10) discloses a multiparticle that contains a core containing a drug, a matrix material having heat melting properties, and a water-swellable swelling agent, in which the core is surrounded by a coating selected from the group consisting of a coating which passes water and substantially does not pass a drug, and a coating having enteric-dissolution resistant properties.

### Citation List

### Patent Literature

Patent Literature 1: WO 02/96392 A
Patent Literature 2: JP 2007-63263 A
Patent Literature 3: JP 2008-214334 A
Patent Literature 4: JP 2008-260712 A
Patent Literature 5: JP 2011-063627 A
Patent Literature 6: JP 2000-191519 A
Patent Literature 7: JP 2011-225468 A
Patent Literature 8: JP H03-130214 A
Patent Literature 9: WO 2012/036078 A
Patent Literature 10: JP 2007-532623 A

### Summary of Invention

### Technical Problem

In the drug described in Patent Literature 1, after the water-soluble polymer has been eluted from the film, fine pores are formed in the film, and the fine pores act as water passages which allow the drug to pass through the film. However, in the film formulated so as to have sufficient drug masking properties or a film having a thickness which allows the film to mask an unpalatable taste sufficiently, water passages which are sufficient for achieving rapid drug release cannot be formed. Accordingly, when the amount of coating is increased to prolong a lag time for achieving the "suppression of the initial drug release", the rapid drug release after the lag time cannot be achieved. In addition, when the amount of a water-soluble polymer in the mixed film is increased to achieve the rapid drug release after the lag time, the suppression of the initial drug release becomes difficult. Thus, it is required that the amount of the coating is increased until a desired lag time is achieved, and thus also the rapid drug release cannot be achieved. In other words, practically, it is almost impossible to achieve both the controlling of the lag time and the rapid dissolution rate of a drug concurrently by coating pharmaceutical composition particles with a mixed film of a water-insoluble substance and a water-soluble substance.

In addition, even by the methods described in Patent Literatures 2 to 5, when the lag time is sufficient, intended rapid drug release cannot be achieved. As described above, by a single-layered coating only, it is difficult to achieve both the sufficient lag time and the rapid drug release.

In addition, by using only a water-soluble substance as described in Patent Literature 6, it is difficult to achieve a prolonged lag time. It is to be noted that Patent Literature 6 describes nothing about lag time control.

In the delayed release granular pharmaceutical composition described in Patent Literature 7, there are problems that preparation of spherical particles is difficult and the yield of particles having a uniform particle diameter is low owing to the method of manufacturing the composition, the method of measuring time of masking of a bitter taste, in which particles are held in the oral cavity for only 30 seconds, is insufficient, and there is almost no reference to lag time. In Patent Literatures 8, 9, and 10, a material practically insoluble in water, which is generally referred to as a disintegrant, is used as a water-swellable substance or a water-swellable polymer. The rapid release preparation described in Patent Literature 8 masks an unpalatable taste for about 30 seconds, which is too short and insufficient. The drug-containing film-coated particles described in Patent Literature 9 are evaluated about an unpalatable taste by a method in which the particles are held in the oral cavity for a short time, that is, 30 seconds. However, this evaluation is insufficient. In addition, Patent Literature 9 does not refer to lag time. Further, in drug-containing film-coated particles in many Examples described in Patent Literature 9, amount drug dissolution after 60 minutes does not reach 90%, and thus the drug-containing film-coated particles do not necessarily have rapid dissolving properties.

As described above, in the conventional bitter taste mask method using inner cores formed of a water-swellable substance such as a disintegrant which is simply swollen with water, and in a bitter taste mask method by a film agent using a mixture including a water-insoluble substance and a water-soluble substance, it is not possible to achieve both a desired lag time and rapid drug release after a lapse of the desired lag time. It is though that this is mainly because when a water-swellable substance such as a disintegrant which is simply swollen with water is used, water-absorbing swelling ability is insufficient and the water-swellable substance is not soluble in water, and thus the drug in the particles spreads outside slowly after the outer layer has been broken.

Accordingly, objects of the present invention are to provide a pharmaceutical composition particle for oral administration which is capable of achieving both masking of an unpalatable taste and improvement in dissolution properties, an orally disintegrating preparation containing the pharmaceutical composition particle, and a method for manufacturing the pharmaceutical composition particle. In other words, objects of the present invention are to provide a pharmaceutical composition particle for oral administration which has a lag time which is sufficiently long to prevent dissolution of a drug in the oral cavity, is capable of performing rapid drug release after the lag time, and is capable of controlling the length of the lag time in accordance with purposes; an orally disintegrating preparation containing the pharmaceutical composition particle; and a method for manufacturing the pharmaceutical composition particle.

### Solution to Problem

The present inventors have made extensive investigations to solve the above problem. As a result, the present inventors have found that a pharmaceutical composition particle having a multi-layer structure including a core particle, as a central part of a particulate composition, containing a water-soluble gelling swelling substance and a drug, in which the core particle is coated with a layer containing a water-insoluble component as a water infiltration quantity controlling layer, in which the coating layer is provided on the outer side of the core particle, has a lag time which is sufficient to avoid any feeling of an unpalatable taste in the mouse and capable of realizing rapid drug release after the lag time. The present inventors further have found that by changing the amount and the component of coating in each coating layer, the length of the lag time can be controlled to be about within 2 to 10 minutes.

A remarkable feature of the present invention is that a particle formed by arranging a gelling swelling substance and a drug in a matrix form is used as a core particle. In a publicly known bitter taste masking fine particle using a water-swellable substance such as a disintegrant which is simply swollen by absorption of water does not achieve sufficient masking of an unpalatable taste as described above. The present inventors have found a method in which a water-soluble gelling swelling substance, which absorbs water, forms a gel, and swells to change into a pasty state, and increases in volume, is used and mixed with a drug to form a particle, which is used as a core particle, and a layer containing a water-insoluble polymer is provided on the core particle as an outer layer.

A detailed mechanism of drug dissolution from a pharmaceutical composition particle according to the present invention is as follows. Water in the oral cavity passes through the outer layer containing a water-insoluble substance and gradually infiltrates into the particle. The water infiltrated into the particle causes a gelling swelling substance in the core to form a gel, swell, and change into a pasty state. While the core particle forms a gel and swells to change into a pasty state, transfer of the water from the inside of the pharmaceutical composition particle to the outside is prevented, and thus the time required for forming a gel and swelling causes a lag time. The gelling swelling substance which has absorbed water forms a highly viscous gel and increases in volume. As a result, the gelling swelling substance pushes and stretches the outer layer, which results in film deformation of the outer layer, for example, the outer layer becomes a thin film, or cracks and a breakage are formed in of the outer layer, and thereby the gelling swelling substance diffuses into water. The gelling swelling substance forms a gel temporarily, thereafter, however, the gel becomes fragile as the amount of water transferred into the particle increases over time, and thus the gelling swelling substance rapidly dissolves in water and diffuses into water. Accordingly, the drug diffuses together with the gelling swelling substance, and rapid release of the drug is achieved.

Based on the above-described findings, the present invention has been made to have the following constitution. In other words, a pharmaceutical composition particle according to the present invention includes: a core particle containing a drug and a water-soluble gelling swelling substance; and an outer layer which contains a water-insoluble substance and constitutes a coating provided on the outer side of the core particle.

As described later in detail, by adjusting a component and the amount of coating of the outer layer and/or a component included in the core particle such as a gelling swelling substance, the lag time can be controlled.

As a result of extensive investigations which have been made by the present inventors, it has been found that the amount of the gelling swelling substance in the core particle does not have a strong influence on the release rate of the drug after the film deformation occurs, for example, the outer layer becomes a thin film, or cracks and breakage of the outer layer are formed. Therefore, it has been found that by increasing the content of the gelling swelling substance in the core particle to a certain extent, a steady lag time can be achieved. On the other hand, it has been found that when water absorption and swelling of the gelling swelling substance and enlargement of the gelling swelling substance occur, the outer layer becomes a thin film or cracks and breakage of the outer layer are formed, and thus even when the amount and the thickness of the outer layer are increased to a certain extent, the drug release rate after the lag time does not decreases.

Thus, for example, when an outer layer which can maintain its shape in the oral cavity for 1 to 2 minutes or longer is formed, a pharmaceutical composition particle does not disintegrate in the oral cavity for at least 1 to 2 minutes, and as a result, an unpalatable taste can be masked for at least 1 to 2 minutes. During this lag time of 1 to 2 minutes, water infiltrates through the outer layer into the particle. Then, the pharmaceutical composition particle moves to the stomach, and thereafter the outer layer becomes a thin film or cracks and breakage of the outer layer are formed by swelling of the core particle within about several to 10 minutes as described above. In this way, both the controlling of the lag time and the rapid drug release can be achieved.

As described above, a pharmaceutical composition particle which is capable of achieving both masking of an unpalatable taste and improvement in dissolution properties, that is, a pharmaceutical composition particle for oral administration which has a lag time which is sufficiently long to prevent dissolution of a drug in the oral cavity, is capable of performing rapid drug release after the lag time, and is capable of controlling the length of the lag time in accordance with purposes can be provided.

In addition, by extensive investigations made by the present inventors, it has been found that when a gelling swelling substance which has a viscosity of 10 mPa·s or more in a 2% aqueous solution at 25°C is used, both the controlling of the lag time and the rapid dissolution rate are achieved more preferably.

Therefore, in the pharmaceutical composition particle according to the present invention, the gelling swelling substance preferably has a viscosity of 10 mPa·s or more in a 2% aqueous solution at 25°C.

In addition, the outer layer of the pharmaceutical composition particle according to the present invention preferably contains a water-insoluble substance in an amount of 60% by weight or more with respect to the total weight of the outer layer.

Herein, swelling power of a gelling swelling substance is defined as follows. When the gelling swelling substance is mixed with water to give a highly viscous and thick malt syrup-like solution having a viscosity at 30°C of 1900 to 2100 mPa·s, the amount of water (part by weight) included per 100 (part by weight) of the gelling swelling substance is defined as the swelling power (S) of the gelling swelling substance. Then, in the pharmaceutical composition particle according to the present invention, the gelling swelling substance contained in the core particle is preferably a gelling swelling substance having a swelling power (S value) of 650 or more.

In addition, in the pharmaceutical composition particle according to the present invention, the amount of coating of the outer layer is preferably 5% by weight or more and 50% by weight or less with respect to the core particle.

An orally disintegrating tablet according to the present invention includes any of the above-described pharmaceutical composition particles.

A method for manufacturing the pharmaceutical composition particle according to the present invention includes: a pulverization step of pulverizing a water-soluble gelling swelling substance so that the pulverized water-soluble gelling swelling substance has an average particle diameter of 15 µm or less; a core particle manufacturing step of mixing the gelling swelling substances pulverized in the pulverization step and a drug to manufacture a core particle; and an outer layer formation step of coating the core particle with a water-insoluble substance on the outer side of the core particle to form an outer layer.

The core particle manufacturing step is preferably a step such as, for example, (1) a step of adding a solution in which a binder is dissolved to a mixture of a pulverized gelling swelling substance and a drug to manufacture a particle using a tumbling granulating apparatus or the like; (2) a step of directly manufacturing a substantially sphere-shaped particle by a splay granulation method using a solution in which a gelling swelling substance and a drug are suspended or dissolved in a solution in which one kind of binder, or two or more kinds of binders, if necessary, are dissolved; (3) a step of spraying and coating, using a tumbling fluidized bed granulating apparatus or the like, an inert spherical particle with a solution in which a pulverized gelling swelling substance and a drug are suspended or dissolved in a solution in which one kind of binder, or two or more kinds of binders, if necessary, are dissolved to manufacture a particle; or the like.

In the method for manufacturing a pharmaceutical composition particle according to the present invention, the a core particle manufacturing step preferably includes a step of suspending the gelling swelling substance pulverized in the pulverization step and a drug in an organic solvent, and the organic solvent is preferably an organic solvent such as ethanol which does not dissolve the gelling swelling substance.

Since a highly viscous gelling swelling substance, for example, having a viscosity of 10 mPa·s or more forms a gel-like or paste-like solution which is harder than thick malt syrup when dissolved in water, the highly viscous gelling swelling substance cannot be used for the manufacture of fine particle cores or coating operation as it is. In addition, since a gelling swelling substance is fibrous, the gelling swelling substance cannot easily be finely pulverized by a commonly used agitator or pulverizer. Thus, the gelling swelling substance is scarcely used as a coating material as particles which are to be suspended in a coating liquid, or scarcely used as an additive agent for granulation. Although a method in which the gelling swelling substance is dissolved in water at an extremely low concentration may be possible, the method requires long time for coating and is not practical. In addition, it has been thought that the gelling swelling substance clings to a particle or a drug, which causes disadvantages such as inhibition of rapid drug release properties of the drug. Therefore, it has been considered to be pharmaceutically extremely difficult to manufacture a substantially sphere-shaped fine particle core using a gelling swelling substance as a main component, or to coat a fine particle with a gelling swelling substance.

The present inventors have found a method for manufacturing a core particle by finely pulverizing a gelling swelling substance so that the pulverized gelling swelling substance has an average particle diameter of 15 µm or less, mixing the finely pulverized gelling swelling substance with a drug, and granulating the mixture using an organic solvent such as ethanol; or a method for manufacturing a core particle including a gelling swelling substance and a drug by suspending or dissolving the finely pulverized gelling swelling substance and a drug in an organic solvent such as ethanol to form a solution, and using the solution as a solution for granulation or coating. In particular, it is considered that when the gelling swelling substance is finely pulverized so that the pulverized gelling swelling substance has an average particle diameter of 15 µm or less, a substantially sphere-shaped particle can be easily obtained, the gelling swelling substance can be coated as a uniform film, and other effects can be exerted, and thus the gelling swelling substance is rapidly dispersed in water after a lag time and the core particle is rapidly exposed to water.

As described above, a method for manufacturing a pharmaceutical composition particle for oral administration which is capable of achieving both masking of an unpalatable taste and improvement in dissolution properties, that is, a pharmaceutical composition particle which has a lag time which is sufficiently long to prevent dissolution of a drug in the oral cavity, is capable of performing rapid drug release after the lag time, and is capable of controlling the length of the lag time in accordance with purposes can be provided.

### Brief Description of Drawings

FIG. 1 is a graph showing results of a dissolution test at a paddle rotation rate of 50 rpm and 100 rpm with respect to fine particles in Example 2.
FIG. 2 is a graph showing results of a dissolution test at a paddle rotation rate of 50 rpm and 100 rpm with respect to fine particles in Example 4.
FIG. 3 is a graph showing results of a dissolution test at pH 1.2 and pH 6.8 with respect to fine particles in Example 2.

### Description of Embodiments

Embodiments of the present invention will be described below.

Herein, a "pharmaceutical composition particle" refers to a drug-containing particulate composition which can be used in various oral dosage forms.

Herein, "suppressing drug release" or "suppressing initial drug release" refers to suppressing a dissolution rate of a drug so as to be 0 to 20% in a dissolution test by a paddle method at 50 rpm according to a dissolution test in the Japanese Pharmacopeia using a test solution which is intended to simulate an intraoral condition. Hereinafter, a period of time during which a drug dissolution rate remains within the above-described range of 0 to 20% is referred to as a "lag time".

When the particle according to the present invention is retained in the oral cavity for a certain period of time, saliva moves through the outer layer into the particle and a gelling swelling substance is swollen. Even when a particle is such a particle that the gelling swelling substance of the particle is softened and gradually dissolved in 900 mL of a dissolution test fluid to release 0 to 20% of a drug in the particle within 2 minutes, in a small amount of saliva present in the oral cavity, the gelling swelling substance only becomes a gel and changes into a clayey state in the outer layer and does not change into a state in which the gelling swelling substance diffuses into the oral cavity, and, of course, a drug in the particle does not diffuse into the oral cavity and a bitter taste is masked in the oral cavity.

According to the findings which have been made by the present inventors, in order to mask an unpalatable taste of a drug for 1 to 2 minutes after oral administration of a pharmaceutical composition particle, a lag time of about 2 minutes is required. Also, in the pharmaceutical composition particle according to the present invention, when the above-described dissolution rate in the dissolution test is satisfied, masking of an unpalatable taste of a drug is achieved. It is to be noted that when a gelling swelling substance is exposed at an outer layer of a particle in a test at 50 rpm, due to the viscosity of the gelling swelling substance, a phenomenon in which the particles aggregate and precipitate on the round-bottom portion of the vessel for the dissolution test and the drug release becomes difficult. With respect to pharmaceutical composition particles which cause the above-described phenomenon, the test was carried out at 100 rpm to avoid the aggregation and the precipitation.

Herein, the expression "rapidly releasing a drug" or "rapid drug release" refers to a drug release condition in which a sufficient drug efficacy can be expected to be achieved. In other words, in a dissolution test using a test solution which is intended to simulate a gastrointestinal tract fluid, it is at least required that a drug dissolution rate at 60 minutes after the beginning of the dissolution test is at least 90%. When a higher rapid release property is required, it is preferred that a drug dissolution rate at 30 minutes after the beginning of the dissolution test is at least 80%. If a drug dissolution rate does not reach the above-mentioned rates in this dissolution test, it means that, depending on a drug, absorption of the drug in an upper part of the gastrointestinal tract is reduced and a sufficient drug efficacy cannot be expected to be achieved.

Herein, the "unpalatable taste" specifically refers to a bitter taste, a rough taste, a harsh taste, an acid taste, an astringent taste, a pungent taste, and the like.

Herein, as the "test solution which is intended to simulate an intraoral condition", according to the knowledge that pH in the oral cavity indicates a weakly acidic condition, the 2nd fluid for dissolution test in the Japanese Pharmacopeia (phosphate buffer solution with pH 6.8) was used. As the "test solution which is intended to simulate a gastrointestinal tract liquid", the "1st fluid for disintegration test in the Japanese Pharmacopeia (hydrochloric acid buffer solution with pH 1.2)" in consideration of a variation in intragastric pH, or the 2nd fluid for dissolution test was used.

A constitution and the like of the pharmaceutical composition particle according to the present invention will be described below.

Herein, a "core particle containing a water-soluble gelling swelling substance and a drug" refers to a particle consisting only of a water-soluble gelling swelling substance and a drug, or a particle including a water-soluble gelling swelling substance, a drug, and an additive or two or more additives. The "core particle containing a water-soluble gelling swelling substance and a drug" also includes a particle in which a core which contains no water-soluble gelling swelling substance and no drug is coated with a water-soluble gelling swelling substance and a drug. As the core particle, two or more kinds of water-soluble gelling swelling substances may be used as a mixture, and a water-soluble gelling swelling substance and a drug may form a plurality of layers which may constitute the core particle.

As a water-soluble gelling swelling substance used in the present invention, it is important that the substance is a substance which absorbs water, forms a gel in a pasty state, and swells. Examples of the gelling swelling substance include carboxymethylcellulose sodium salt, polyethylene oxide, sodium polyacrylate, sodium alginate, propylene glycol alginate, xanthan gum, carrageenan, guar gum, tara gum, pectin, hydroxypropylmethylcellulose, hydroxypropylcellulose, hydroxyethylcellulose, methylcellulose, a carboxyvinyl polymer, locust bean gum, tamarind seed gum, gum arabic, karaya gum, agar, gelatin, and polyvinyl alcohol; or a copolymer containing polyvinyl alcohol as a part of the copolymer. One kind of these gelling swelling substances, or two or more kinds of these gelling swelling substances are used to manufacture a core particle.

These gelling swelling substances dissolve in water and change into a pasty state to form a highly viscous solution. Thus, it is basically impossible to form a spherical granular particle of a gelling swelling substance and a drug formulation by a wet granulation method or a coating method which uses water as a solvent. Therefore, it has been found that the above-described problem can be solved by, for example, a method in which a gelling swelling substance is pulverized so that the pulverized gelling swelling substance has an average particle diameter of 15 µm or less, preferably 10 µm or less, and the pulverized gelling swelling substance is mixed with a drug, and the mixture is granulated using an organic solvent such as ethanol to manufacture a core particle; or by suspending or dissolving the pulverized gelling swelling substance and a drug in an organic solvent such as ethanol to form a solution, and using the solution as a solution for granulation or coating, which is used for the manufacture of a core particle including a gelling swelling substance and a drug. At this time, water may be freely blended into the organic solvent as long as the suspension is not impaired. In addition, for the purpose of improving adhesion between a gelling swelling substance and a drug, binders such as hydroxypropylcellulose, hydroxypropylmethylcellulose, polyvinylpyrrolidone, copolyvidone, macrogol, ethylcellulose, polyvinylacetal diethylaminoacetate, hydroxypropylmethylcellulose, and hypromellose phthalate, which are widely used as binders for granulation or coating, may be freely formulated or dissolved together, that is, may be freely used together. It is to be noted that a method for manufacturing the core particle is not limited as long as a core particle can be manufactured with a gelling swelling substance and a drug formulation.

In addition, for the purpose of controlling dissolution rate or the like of the gelling swelling substance used for the core particle, saccharides or sugar alcohols such as mannitol, erythritol, and xylitol, or organic acids such as citric acid, tartaric acid, and malic acid may be freely blended into the core particle.

Viscosities and swelling power of the above-mentioned gelling swelling substances considerably differ among species and grades. Accordingly, the amount of the gelling swelling substance included in the core particle cannot be simply determined. However, by way of illustration, the amount of the gelling swelling substance included in the core particle which is manufactured by blending the gelling swelling substance and a drug and directly granulating the blend is preferably 20% or more, and more preferably 40% or more. In addition, when a core particle is produced by coating a core containing no gelling swelling substance and no drug with a gelling swelling substance and a drug, the amount of coating of the gelling swelling substance is preferably 5% by weight or more with respect to the core containing no gelling swelling substance and no drug, and more preferably 10% by weight or more; and in addition, preferably 75% by weight or less, and more preferably 60% by weight or less. If the amount of coating is smaller than 5% by weight, there is a possibility that a sufficiently long lag time cannot be achieved.

Herein, the "outer layer" refers to a coating layer including one kind of water-insoluble substance or two or more kinds of water-insoluble substance, and the outer layer may contain one kind of water infiltration modifier or two or more kinds of water infiltration modifiers. The outer layer is coated as an outer layer of the core particle. The outer layer controls the rate of infiltration of water into a pharmaceutical composition particle, and thereby controls the rate of water absorption, gelation, and swelling of the core particle to achieve a lag time.

The outer layer may be directly applied onto a core particle, or the outer layer may be applied to a core particle after the core particle has been coated with a component which does not constitute an obstacle to providing lag time and rapid drug release after the lag time as a single-layered coating layer or a two-or-more-layered coating layer. Onto the outer layer, a component which does not constitute an obstacle to providing lag time and rapid drug release after the lag time can be applied as a single-layered coating layer or a two-or-more-layered coating layer. In addition, since the purpose of the outer layer is to control the rate of infiltration of water, the outer layer may be a single layer or a multi-layered layer including two or more layers in accordance with purposes of the control.

The water-insoluble substance used for forming the outer layer is one of essential components provided in the outer layer to control the rate of infiltration of water, and has a solubility such as slightly soluble, very slightly soluble, or practically insoluble in water. Specific examples of the water-insoluble substance include, but are not limited to, ethylcellulose; acetylcellulose; cellulose acetate phthalate; carboxymethylethylcellulose; hydroxypropylmethylcellulose acetate succinate; hydroxypropylmethylcellulose phthalate; a dimethylaminoethyl methacrylate-methyl methacrylate copolymer; a methyl acrylate-methacrylic acid copolymer; an ethyl acrylate-methyl methacrylate copolymer dispersion liquid; an aminoalkyl methacrylate copolymer RS; a dry methacrylic acid copolymer LD; an aminoalkyl methacrylate copolymer E; a methacrylic acid copolymer L; a methacrylic acid copolymer LD (an aqueous dispersion liquid); a methacrylic acid copolymer S; polyvinylacetal diethylaminoacetate; dried milky white lac; shellac; zein; higher fatty acids such as stearic acid; higher alcohols such as cetanol and stearyl alcohol; a low melting point substance having a melting point of 30 to 120°C such as carnauba wax, beeswax, and paraffin; an ester of a higher fatty acid and a polyhydric alcohol such as a sucrose fatty acid ester; fat obtained by hydrogenation of oil such as castor wax; and lubricants such as magnesium stearate, synthetic wax, and talc. The water-insoluble substances may be used alone or in proper combination of two or more. In addition, plasticizers such as castor oil, dibutyl phthalate, and triethyl citrate may be freely mixed and used.

In addition, when the outer layer is formed, as a water infiltration modifier, a water-soluble substance, a hydrophilic substance, or the like can be blended into the water-insoluble substance. Herein, the water infiltration modifier refers to a component which is blended together with the a water-insoluble substance into the outer layer in order to adjust the rate of infiltration of water and the amount of infiltration of water, and the water infiltration modifier is a component which is freely soluble in water and a component such as a disintegrant which is hydrophilic and very permeable to water. Specific examples of the water infiltration modifier in the outer layer according to the present invention include, but are not limited to, pregelatinized starch; casein sodium; a carboxyvinyl polymer; sodium carboxymethyl starch; a sucrose fatty acid ester; hydroxypropylcellulose; hydroxypropylmethylcellulose; methylcellulose; hydroxyethylcellulose; pullulan; polyvinylpyrrolidone; copolyvidone; polyoxyethylene-polyoxypropylene glycol; a polyvinyl alcohol-polyethylene glycol graft copolymer; polyvinyl alcohol; macrogol; polyethylene oxide; amino acids such as glycine and alanine; sweeteners such as glycyrrhizic acid; saccharides such as dextrin and lactose; sugar alcohols such as mannitol and xylitol; crystalline cellulose; crospovidone; and triethyl citrate. The water infiltration modifiers may be used alone or in proper combination of two or more.

As the composition ratio of the water-insoluble substance and the water infiltration modifier in the outer layer according to the present invention, in accordance with purposes with respect to physical properties and an absorption site of a drug, types of preparations, and the like, a ratio suitable for achieving the purposes is selected. It is to be noted that, in the pharmaceutical composition particle according to the present invention, when water permeability of the outer layer is high (when the amount of a water-infiltration substance which is included together with the water-insoluble substance is large), the gelling swelling substance blended in the core particle may be rapidly exposed through fine pores of the outer layer in the oral cavity, and the particles may aggregate in the oral cavity and may adhere to the oral mucosa due to the viscosity of the gelling swelling substance. Accordingly, the content of the water-insoluble substance in the outer layer is preferably 60% by weight or more. In addition, when the proportion of the water-insoluble substance is lower than 30% by weight, there is a possibility that the rate of infiltration of water into the pharmaceutical composition particle cannot be sufficiently controlled, and a sufficiently long lag time cannot be achieved.

As the amount of coating of the outer layer according to the present invention, an amount suitable for achieving the object of the present invention is selected. Specifically, the amount of coating is preferably 5% by weight or more with respect to the core particle, and particularly preferably 7% by weight or more, and in addition, preferably 50% by weight or less, and particularly preferably 30% by weight or less. When the amount of coating is less than 5% by weight, there is a possibility that the coating on the surface of the pharmaceutical composition particle may not be uniform, and in addition, there is a possibility that the rate of infiltration of water into the pharmaceutical composition particle cannot be sufficiently controlled because the outer layer is very thin, and there is a possibility that a sufficiently long lag time cannot be achieved, and adhesion of the particle to the oral cavity may occur. In addition, if the amount of coating is too large, rapid drug release after the lag time may not be achieved.

The drug used in the present invention is not particularly limited as long as the drug is an active component which is therapeutically or prophylactically effective. Examples of the pharmaceutically active component include a hypnotic and sedative, a sleep inducing drug, a migraine drug, an anxiolytic, an antiepileptic drug, an antidepressant drug, an antiparkinsonian drug, a psychoneurotic drug, a drug for the central nervous system, a local anesthetic, a skeletal muscle relaxant, an autonomic agent, an antipyretic analgesic antiphlogistic, an antispasmodic, an antidizziness drug, a cardiotonic drug, an antiarrhythmic agent, a diuretic, a hypotensive drug, a vasoconstrictor, a vasodilator, a cardiovascular agent, a hypolipidemic drug, a respiratory stimulant, an antitussive drug, an expectorant, an antitussive expectorant agent, a bronchodilator, an antidiarrheal drug, an intestinal regulator, an antiulcer drug, stomachics and digestives, an antacid, a cathartic drug, a choleretic drug, an agent for digestive organs, an adrenal hormone preparation, a hormone drug, an agent for urinary organs, a vitamin preparation, a hemostatic drug, a drug for liver diseases, a therapeutic drug for gout, an antidiabetic drug, an anti-histamine drug, an antibiotic drug, an antibacterial drug, an antineoplastic drug, a chemotherapeutic drug, a multi-ingredient cold medication, a nutritional fortification health drug, and a drug for osteoporosis.

The amount of a drug included in the pharmaceutical composition particle is not particularly limited. The amount of a drug included is preferably 0.5% by weight or more with respect to the total amount of the pharmaceutical composition particle, and in addition, preferably 80% by weight or less, particularly preferably 70% by weight or less, and further preferably 60% by weight or less. However, the above-mentioned amount of a drug included is merely an example which is applicable to the present invention, and should not be construed as limiting.

The pharmaceutical composition particle according to the present invention preferably has a particle diameter as a length of the longest axis of 2 mm or less. When the shape of the pharmaceutical composition particle can be approximated by a sphere, the pharmaceutical composition particles according to the present invention preferably has an average particle diameter of 2 mm or less. In addition, when the pharmaceutical composition particle has a shape other than sphere, the pharmaceutical composition particles according to the present invention has an average length of the longest axes of 2 mm or less.

The pharmaceutical composition particle according to the present invention in which an unpalatable taste is masked is particularly useful as a fine particle for an orally disintegrating tablet which is retained in the oral cavity for a relatively long time. When the pharmaceutical composition particle according to the present invention is contained in an orally disintegrating tablet, in order to reduce a rough feeling in the oral cavity, the pharmaceutical composition particle is preferably prepared so as to have an average particle diameter of 350 µm or less. The pharmaceutical composition particles more preferably have an average particle diameter of 50 to 350 µm, and still more preferably 70 to 300 µm.

It is needless to say that the pharmaceutical composition particle according to the present invention can be manufactured by using various pharmaceutical additives which are commonly used as additives. Examples of the pharmaceutical additives include, but are not limited to, a taste masking agent, a sweetener, a flavoring agent, a coloring agent, a stabilizing agent, an antioxidant, a pH adjustor, a solubilizing agent, a solubilizing agent, a fluidizer, and a buffer.

The pharmaceutical composition particle according to the present invention can be blended as it is in an amount required, and the blend can be prepared in variety forms of pharmaceutical compositions for oral administration. Herein, preparations refer to powder, granules, tablets, troches, dry syrup preparations, and the like. In some cases, the powder or the granule can be prepared by simply blending the fine particles for formulation and mixing the blend. When an orally disintegrating tablet is prepared, it is required that the fine particles for formulation are blended, and an ingeniously contrived formulation method is used.

Next, a method for manufacturing the pharmaceutical composition particle according to the present invention will be described.

The pharmaceutical composition particle according to the present invention is manufactured by coating a core particle containing a gelling swelling substance and a drug with an outer layer. As the core particle, by using a publicly known technique, a particle consisting only of a gelling swelling substance and a drug can be manufactured and the resulting particle can be used, or a particle including a gelling swelling substance, a drug, and an additive or two or more additives can be manufactured and the resulting particle can be used. The particle including a gelling swelling substance, a drug, and additives can be manufactured by, for example, mixing a gelling swelling substance, a drug, and suitable excipients (e.g., crystalline cellulose, lactose, corn starch, or the like), adding a solution in which a binder is dissolved if necessary, granulating, performing particle size regulation, and drying. Also, a method in which a gelling swelling substance is continuously splayed with a solution in which a drug is dissolved or suspended together with a binder or the like by means of fluidized bed can be used In addition, by using a fluidized bed coating apparatus, the pharmaceutical composition particle can be manufactured by spraying additive particles (e.g., crystalline cellulose (particles), purified white sugar spherical particles, mannitol spherical particles, or the like), which can be suitable cores, with a liquid in which a gelling swelling substance, a drug, a binder, and the like are dissolved or suspended. It is needless to say that the core particle according to the present invention can be manufactured by various other methods such as a splay granulation method, a melt granulation method, a spray chilled method, a dry granulation method, or the like.

When the pharmaceutical composition particle is used for the manufacture of an orally disintegrating tablet, it is generally desired that the pharmaceutical composition particle has an average particle diameter of approximately 100 to 200 µm. When a core particle is manufactured by coating substantially sphere-shaped particles of crystalline cellulose (particles) or the like having an average particle diameter of 75 to 150 µm with a gelling swelling substance and a drug as an outer layer, in order to maintain the spherical shape of the substantially sphere-shaped core particle while the core particle is coated with the gelling swelling substance and the drug to form the pharmaceutical composition particle having an average particle diameter of approximately 100 to 200 µm, it is required that the average particle diameter of a substance (e.g., a gelling swelling substance) which is suspended in a coating solution is one-tenth or less of the diameter of the particle to be coated. Accordingly, the average particle diameter of a gelling swelling substance which is suspended in a coating solution and used for coating is preferably 15 µm or less, more preferably 10 µm or less, and most preferably 7 µm or less. This also applies to other cases such as a case in which the substantially sphere-shaped core particle is manufactured by a direct granulation method. Herein, the average particle diameter is as measured by using a laser diffraction/scattering particle size distribution analyzer.

However, since the gelling swelling substance is fibrous, the gelling swelling substance is hardly pulverized and cannot be easily micronized. Therefore, the present inventors have made extensive investigations regarding a pulverization method. As a result, the present inventors have found a method in which the gelling swelling substance can be effectively pulverized into particles having an intended particle diameter by using a jet mill. In addition, the present inventors have found that the gelling swelling substance can be effectively pulverized also by a wet crushing method.

As a method for coating a core particle with an outer layer, it is preferred that a method for coating by using a machine which is widely used for coating operation such as a fluidized bed coating apparatus, a tumbling coating apparatus, a centrifugal tumbling coating apparatus, or the like is used. For example, a required amount of liquid containing a coating component may be sprayed by means of a spray gun while the core particle containing a gelling swelling substance and a drug flows in a tumbling fluidized bed coating apparatus. The liquid containing a coating component is prepared by dissolving or dispersing an essential component in a solvent such as water, ethanol, or methanol. The solvents may be used as a mixture prepared by properly mixing the solvents, or water may be used alone as the solvent. In addition, it is needless to say that the method for coating of these layers is not limited to a wet method.

As described above, the method for manufacturing a pharmaceutical composition particle according to the present invention include a pulverization step of pulverizing the gelling swelling substance so that the pulverized gelling swelling substance has an average particle diameter of 15 µm or less; a core particle manufacturing step of mixing the gelling swelling substance pulverized in the pulverization step and a drug to manufacture a core particle; and an outer layer formation step of coating the core particle with a water-insoluble substance on the outer side of the core particle to form an outer layer. The core particle manufacturing step preferably include a step of suspending the gelling swelling substance pulverized in the pulverization step and the drug in an organic solvent, and the step is preferably a core particle manufacturing step in which the core particle is manufactured by performing granulation or layering using the gelling swelling substance pulverized in the pulverization step and the drug.

In addition, when an organic solvent is used in the core particle manufacturing step, the organic solvent is preferably ethanol.

An orally disintegrating tablet containing the pharmaceutical composition particle according to the present invention will be described below.

The orally disintegrating tablet according to the present invention refers to a preparation which is a tablet or the like which disintegrates in the oral cavity within a certain time, preferably within 1 minute, and more preferably 45 seconds. Examples of the orally disintegrating tablet include orally disintegrating tablets containing pharmaceutical composition particles disclosed in JP 2012-240917 A, JP 4019374 B, JP 3746167 B, or the like. As in the orally disintegrating tablets described above, the pharmaceutical composition particle according to the present invention can be formulated together with appropriate excipients, disintegrants, binders, lubricants, and the like to afford an orally disintegrating tablet.

Specific examples of the manufacturing method include various manufacturing methods including (1) a method for manufacturing a tablet by mixing the pharmaceutical composition particles as it is with a sugar or a sugar alcohol and a selected disintegrant, and performing pressurizing and compressing; (2) a method for manufacturing a tablet by mixing the pharmaceutical composition particles together with a sugar or a sugar alcohol and a selected disintegrant, granulating the resulting mixture using a binder solution to form a particle, mixing the resulting particle with other suitable additive agents for tablets, and performing pressurizing and compressing; and (3) a method for manufacturing a tablet by mixing the pharmaceutical composition particles with a saccharide having low moldability, spraying the resulting mixture with a saccharide having high moldability as a binder to form a coating and/or granules, performing low-pressure compression molding, and thereafter performing humidification and drying. It is to be noted that in an orally disintegrating tablet blended with the pharmaceutical composition particles, special consideration for destruction of the particles, hardness of the tablet, disintegrating properties, content uniformity, and the like is required. Further, the method for manufacturing an orally disintegrating tablet should not be limited to the methods described above, and any method including a molding method and a wet-molding drying method can be used for tableting.

The amount of the fine particles for formulation, that is, the pharmaceutical composition particles included in a tablet is preferably, but not limited to, 5% by weight or more and 85% by weight or less with respect to the weight of the tablet, more preferably 5% by weight or more and 70% by weight or less, and still more preferably 10% by weight or more and 70% by weight or less. When the mount of the pharmaceutical composition particles included exceeds 85% by weight, there is a possibility that strength and dissolution properties as a tablet, in particular, as an orally disintegrating tablet cannot be achieved.

The orally disintegrating tablet according to the present invention can be manufactured by blending a general additive agent which is commonly used for the manufacture of tablets in addition to the pharmaceutical composition particle according to the present invention. As the excipient, a sugar or a sugar alcohol such as mannitol, erythritol, maltitol, or lactose, crystalline cellulose, calcium hydrogen phosphate, and the like can be used. The binder is not particularly limited as long as the binder is an ordinary binder such as corn starch, polyvinylpyrrolidone, copolyvidone, hydroxypropylcellulose, polyvinyl alcohol, and the like. As the disintegrant, a generally used disintegrant such as carmellose, crospovidone, corn starch, partially pregelatinized starch, carmellose calcium, croscarmellose sodium, low substituted hydroxypropylcellulose, and the like can be used. In the preparation, a sweetener, a taste masking agent, and the like can be freely blended to improve the feeling in taking the preparation.

The present invention is summarized as described below.
(1) The pharmaceutical composition particle according to the present invention includes: a core particle containing a water-soluble gelling swelling substance and a drug; and an outer layer which contains a water-insoluble substance and constitutes a coating provided on the outer side of the core particle.
(2) In the pharmaceutical composition particle according to the above-described (1), the gelling swelling substance preferably has a viscosity of 10 mPa·s or more in a 2% aqueous solution at 25°C.
(3) In the pharmaceutical composition particle according to the above-described (1) or (2), the outer layer preferably contains the water-insoluble substance in an amount of 60% by weight or more with respect to the total weight of the outer layer.
(4) In the pharmaceutical composition particle according to any of the above-described (1) to (3), the gelling swelling substance preferably has a swelling power (S) of 650 or more.
(5) In the pharmaceutical composition particle according to any of the above-described (1) to (4), the amount of coating of the outer layer is preferably 5% by weight or more and 50% by weight or less with respect to the core particle.
(6) An orally disintegrating tablet according to the present invention contains the pharmaceutical composition particle according to any of the above-described (1) to (5).
(7) A method for manufacturing the pharmaceutical composition particle according to the present invention includes: a pulverization step of pulverizing a water-soluble gelling swelling substance so that the pulverized water-soluble gelling swelling substance has an average particle diameter of 15 µm or less; a step of manufacturing a core particle by mixing the gelling swelling substance pulverized in the pulverization step and a drug; and an outer layer formation step of coating the core particle with a water-insoluble substance on the outer side of the core particle to form an outer layer.
(8) In the method for manufacturing according to the above-described (7), the core particle manufacturing step preferably includes a step of suspending the gelling swelling substance pulverized in the pulverization step and the drug in an organic solvent, and the organic solvent is preferably ethanol.

### EXAMPLES

The present invention will be more specifically described below with reference to examples, but the present invention is not limited to the examples.

Preparation raw materials used in experiments are as follows: hydroxypropylmethylcellulose 2910 (TC-5E, viscosity: 3 mPa·s, Shin-Etsu Chemical Co., Ltd.), hydroxypropylmethylcellulose 2910 (TC-5R, viscosity: 5.8 mPa·s, Shin-Etsu Chemical Co., Ltd.), hydroxypropylmethylcellulose 2910 (TC-5S, viscosity: 15.2 mPa·s, Shin-Etsu Chemical Co., Ltd.), carmellose sodium (CELLOGEN F-5A, viscosity: 4 mPa·s, DKS Co. Ltd.), carmellose sodium (CELLOGEN F-7A, viscosity: 15 mPa·s, DKS Co. Ltd.), carmellose sodium (CELLOGEN PR-S, viscosity: 28 mPa·s, DKS Co. Ltd.), carmellose sodium (CELLOGEN F-SC, viscosity: 400 mPa·s, DKS Co. Ltd.), sodium alginate (KIMICAALGIN IL-6, viscosity: 67 mPa·s*), KIMICA Corporation), xanthan gum (KELTROL CG, viscosity (in KCl): 600 mPa·s or more*), Sansho Co., Ltd.), hydroxypropylcellulose (HPC-L, 7.9 mPa·s, Nippon Soda Co., Ltd.), low substituted hydroxypropylcellulose (L-HPC NBD-020, Shin-Etsu Chemical Co., Ltd.), polyvinylpyrrolidone (PVP-K 30, viscosity: 3 mPa·s or less, BASF Japan Ltd.), ethylcellulose (ETHOCEL 7, The Dow Chemical Company), crystalline cellulose (particles) (CELPHERE CP102, Asahi Kasei Chemicals Corporation), polyvinylacetal diethylaminoacetate (AEA, Mitsubishi-Chemical Foods Corporation), hydroxypropylmethylcellulose acetate succinate (AQOAT AS-MG, Shin-Etsu Chemical Co., Ltd.), D-mannitol (160C, Roquette Japan K.K.), crystalline cellulose (KG-802, Asahi Kasei Chemicals Corporation), crospovidone (Polyplasdone XL-10, Ashland), magnesium stearate (Taihei Chemical Industrial Co., Ltd.). It is to be noted that the viscosity marked with an asterisk "*" is a viscosity in a 1% solution at 25°C, and others are viscosities in 2% solutions at 25°C.

The following substances which were suspended in a solution in the following experiments were pulverized by using a jet mill so as to have an average particle diameter of 7 µm or less. The average particle diameters of the pulverized substances were as follows: CELLOGEN F-5A: 5.5 µm, CELLOGEN PR-S: 4.7 µm, CELLOGEN F-SC: 6.5 µm, and KELTROL CG: 3.1 µm.

### [Experimental Example 1]

Various gelling swelling substances were used. A gelling swelling substance and water were mixed, and the amount of water included per 100 parts by weight of the gelling swelling substance when the mixture became a viscous solution having a viscosity at 30°C of 1900 to 2100 mPa·s was defined and designated as swelling power (S) of the gelling swelling substance. The obtained S values were as follows:
TC-5E: 317, TC-5R: 514, TC-5S: 931, CELLOGEN F-5A: 525, CELLOGEN F-7A: 953, CELLOGEN PR-S: 1,011, CELLOGEN F-SC: 2,074, KIMICA ALGIN IL-6: 2,226, KELTROL CG: 5,456, HPC-L: 590, and PVP-K 30: 110.

With respect to CELLOGEN F-7A having an S value of 953, since a solution in which 5 g of CELLOGEN F-7A and 45 mL of ethanol were mixed and suspended had a volume of 49.3 mL, the volume of 5 g of CELLOGEN F-7A corresponded to 4.3 mL. Therefore, an aqueous solution in which 100 g of CELLOGEN F-7A (corresponding to a volume of 86 ml) was mixed with 953 g, which corresponded to the S value, of water had a volume of 1,039 mL, a viscosity of 1900 to 2100 mPa·s, and a water absorption swelling rate of 12.1 times In addition, the same experiment was carried out using HPC-L. The volume of 5 g of HPC-L corresponded to 4.2 mL. Therefore, an HPC-L aqueous solution in which 100 g of HPC-L (corresponding to a volume of 84 mL) was mixed with 590 g, which corresponded to the S value, of water had a volume of 674 mL, a viscosity of 1900 to 2100 mPa·s, and a water absorption swelling rate of 8.0 times. In addition, water absorption swelling rates of other gelling swelling substances were calculated, and the results were as follows: TC-5E: 4.7 times, TC-5R: 7.4 times, CELLOGEN PR-S: 12.5 times, and CELLOGEN F-SC: 25.7 times.

### [Test Example 1]

With respect to pharmaceutical composition particles manufactured by using various core particles (Examples 1 and 2, and Comparative Examples 1 to 3), a dissolution test and evaluation of masking properties of an unpalatable taste were performed.

### [Example 1]

In a solution in which 14.4 g of HPC-L was dissolved in a mixed solution of 162 g of purified water and 648 g of ethanol, 18 g of ambroxol hydrochloride (average particle diameter: about 3 µm) and 57.6 g of CELLOGEN PR-S were suspended to prepare a layering solution. In a mixed solution of 291.6 g of ethanol and 32.4 g of purified water, 32.4 g of ETHOCEL 7 and 3.6 g of TC-5E were dissolved to prepare an outer layer solution.

A tumbling fluidized bed coating granulating machine (model: MP-01, manufactured by Powrex Corporation) was charged with 180 g of crystalline cellulose particles (CELPHERE CP-102) having an average particle diameter of 120 µm. The crystalline cellulose was sprayed and coated with 900 g of the layering solution with agitation and fluidization, dried, and then sifted through a 42-mesh sieve and a 150-mesh sieve to afford drug/gelling agent layering particles (core particles).

Next, a tumbling fluidized bed coating granulating machine (model: MP-01) was charged with 180 g of the drug/gelling agent layering particles. The drug/gelling agent layering particles were sprayed and coated with 360 g of the outer layer solution with agitation and fluidization, and dried to afford outer layer-coated particles (pharmaceutical composition particles) of Example 1.

### [Example 2]

Outer layer-coated particles (pharmaceutical composition particles) of Example 2 were obtained by the same way as in Example 1, except that CELLOGEN F-SC was used instead of carmellose sodium (CELLOGEN PR-S) used in Example 1.

### [Comparative Example 1]

Outer layer-coated particles (pharmaceutical composition particles) of Comparative Example 1 were obtained by the same way as in Example 1, except that CELLOGEN F-5A was used instead of CELLOGEN PR-S in Example 1.

### [Comparative Example 2]

A sample was prepared by using HPC-L instead of carmellose sodium used in Example 1 and Example 2. In a solution in which 72 g of HPC-L was dissolved in a mixed solution of 342 g of purified water and 1,368 g of ethanol, 18 g of ambroxol hydrochloride (average particle diameter: about 3 µm) was suspended to prepare a layering solution. In a mixed solution of 291.6 g of ethanol and 32.4 g of purified water, 32.4 g of ETHOCEL 7 and 3.6 g of TC-5E were dissolved to prepare an outer layer solution.

A tumbling fluidized bed coating granulating machine (model: MP-01, manufactured by Powrex Corporation) was charged with 180 g of CELPHERE CP-102. The CELPHERE CP-102 was sprayed and coated with 1,800 g of the layering solution, dried, and then sifted through a 42-mesh sieve and a 150-mesh sieve to afford drug/gelling agent layering particles (core particles).

Next, a tumbling fluidized bed coating granulating machine (model: MP-01) was charged with 180 g of the drug/gelling agent layering particles. The drug/gelling agent layering particles were sprayed and coated with 360 g of the outer layer solution with agitation and fluidization, and dried to afford outer layer-coated particles (pharmaceutical composition particles) of Comparative Example 2.

### [Comparative Example 3]

Outer layer-coated particles of Comparative Example 3 were produced by the same way as in Example 1 and Example 2, except that L-HPC (NBD020), which was a water-insoluble water-swellable substance widely used as a disintegrant, was used instead of CELLOGEN in Example 1 and Example 2.

With respect to particles obtained in Examples 1 and 2 and Comparative Examples 1 to 3, the following dissolution test and evaluation of masking properties an unpalatable taste were performed.

### [Dissolution Test]

A dissolution test was performed according to the Japanese Pharmacopoeia Method 2 using outer layer-coated particles containing 20 mg of ambroxol hydrochloride by means of an automatic 6-vessel dissolution test apparatus (manufactured by Toyama Sangyo Co., Ltd.). As a test solution, 900 mL of the 2nd fluid for dissolution test in the Japanese Pharmacopeia was used. The paddle rotation rate was 50 rpm or 100 rpm. The results of the dissolution test are shown in Table 1.

**[Table 1]**

| | Paddle rotation rate (rpm) | Dissolution rate (%) | | | | | |
|---|---|---|---|---|---|---|---|
| | | 2 minutes | 5 minutes | 15 minutes | 30 minutes | 45 minutes | 60 minutes |
| Example 1 | 50 | 0.2 | 6.9 | 61.2 | 87.3 | 93.8 | 94.1 |
| Example 2 | 100 | 4.9 | 33.5 | 80.1 | 87.1 | 91.5 | 93.9 |
| Comparative Example 1 | 50 | 1.4 | 11.6 | 30.3 | 47.2 | 59.8 | 69.0 |
| Comparative Example 2 | 50 | 1.2 | 10.5 | 28.8 | 42.9 | 54.9 | 63.5 |
| Comparative Example 3 | 50 | 12.1 | 36.5 | 56.3 | 71.5 | 77.0 | 78.7 |

### [Evaluation of Masking Properties of Unpalatable Taste]

The taste of outer layer-coated particles containing ambroxol hydrochloride and the taste of ambroxol hydrochloride bulk powder were evaluated according to the following evaluation method and the following evaluation criteria. The results are shown in Table 2.

### <Evaluation Method>

Four healthy male human subjects (panelist 1 to panelist 4) took ambroxol hydrochloride-containing film coated particles in an amount equivalent to 15 mg of ambroxol hydrochloride and held the particles in the mouth for 2 minutes, and thereafter spat the particles. An unpalatable taste was evaluated at 1 minute or at 2 minutes after the particles were taken.

### <Evaluation Criteria of Unpalatable Taste>

-: No unpalatable taste was felt.
±: Unpalatable taste was felt slightly, but acceptable.
+: Unpalatable taste was felt.
++: Unpalatable taste was felt strongly.

**[Table 2]**

| | | Example | | Comparative Example | | |
|---|---|---|---|---|---|---|
| | | 1 | 2 | 1 | 2 | 3 |
| 1 minute | Panelist 1 | - | - | - | - | - |
| | Panelist 2 | - | - | - | - | - |
| | Panelist 3 | - | - | - | - | - |
| | Panelist 4 | - | - | - | - | - |
| 2 minutes | Panelist 1 | - | - | - | - | ± |
| | Panelist 2 | - | - | - | - | - |
| | Panelist 3 | - | - | - | - | - |
| | Panelist 4 | - | - | - | - | - |

With respect to ambroxol hydrochloride bulk powder, all of the panelists felt the unpalatable taste strongly as indicated by "++" at 1 minute and thereafter.

With respect to all of the samples in Examples 1 and 2, even at 2 minutes after the particles were taken in the mouth, no unpalatable taste was felt, which means that a clear effect of suppression of the unpalatable taste was obtained. In addition, in the dissolution test, the dissolution rate reached 80% or more at 15 to 30 minutes after the beginning of the dissolution test, which shows that the samples had such drug release properties that rapid development of sufficient drug efficacy could be expected. With respect to Example 2, as described in [Test Example 3] below, since obvious particle aggregation occurred in the dissolution test vessel at 50 rpm, the test was performed at 100 rpm.

On the other hand, with respect to pharmaceutical composition particles in Comparative Examples 1 and 2 which were manufactured by using CELLOGEN F-5A and HPC-L, which had a weak water-absorbing swelling power, as gelling swelling substances, although no unpalatable taste was felt even after 2 minutes, the gelling swelling power was weak and the ability to denature the outer layer was weak. Thus, in the dissolution test, since the drug release rate was approximately 70% even after 60 minutes, it was found that development of sufficient drug efficacy could not be expected. In addition, with respect to particles in Comparative Example 3 which were manufactured by using a water-insoluble water-swellable substance instead of the gelling swelling substance, the unpalatable taste was masked. However, the ability to denature the outer layer was weak and the diffusion ability was weak because the particles were insoluble in water. Thus, in the dissolution test, drug release rate did not reach 80% even after 60 minutes, and it was found that development of sufficient drug efficacy could not be expected. With respect to particles in Comparative Examples 1 to 3, particle aggregation did not occur in the dissolution test vessel during the dissolution test.

### [Test Example 2]

Experiments were performed by using core particles containing a different drug or a different gelling swelling substance.

### [Example 3]

In a solution in which 14.4 g of HPC-L was dissolved in a mixed solution of 150 g of purified water and 660 g of ethanol, 18 g of sertraline hydrochloride (average particle diameter: about 3 µm) and 57.6 g of CELLOGEN PR-S were dissolved or suspended to prepare a layering solution. In a mixed solution of 291.6 g of ethanol and 32.4 g of purified water, 32.4 g of ETHOCEL 7 and 3.6 g of TC-5E were dissolved to prepare an outer layer solution.

A tumbling fluidized bed coating granulating machine (model: MP-01, manufactured by Powrex Corporation) was charged with 180 g of CELPHERE CP-102. The CELPHERE CP-102 was sprayed and coated with 900 g of the layering solution with agitation and fluidization, dried, and then sifted through a 42-mesh sieve and a 150-mesh sieve to afford drug/gelling agent layering particles (core particles).

Next, a tumbling fluidized bed coating granulating machine (model: MP-01) was charged with 180 g of the drug/gelling agent layering particles. The drug/gelling agent layering particles were sprayed and coated with 360 g of the outer layer solution with agitation and fluidization, and dried to afford outer layer-coated particles (pharmaceutical composition particles) of Example 3.

### [Example 4]

In a solution in which 4.8 g of HPC-L was dissolved in a mixed solution of 54 g of purified water and 216 g of ethanol, 18 g of levocetirizine hydrochloride (average particle diameter: about 3 µm) and 7.2 g of KELTROL CG were suspended to prepare a layering solution. In a mixed solution of 291.6 g of ethanol and 32.4 g of purified water, 32.4 g of ETHOCEL 7 and 3.6 g of TC-5E were dissolved to prepare an outer layer solution.

A tumbling fluidized bed coating granulating machine (model: MP-01, manufactured by Powrex Corporation) was charged with 180 g of CELPHERE CP-102. The CELPHERE CP-102 was sprayed and coated with 300 g of the layering solution with agitation and fluidization, dried, and then sifted through a 42-mesh sieve and a 150-mesh sieve to afford drug/gelling agent layering particles (core particles).

Next, a tumbling fluidized bed coating granulating machine (model: MP-01) was charged with 180 g of the drug/gelling agent layering particles. The drug/gelling agent layering particles were sprayed and coated with 360 g of the outer layer solution with agitation and fluidization, and dried to afford outer layer-coated particles (pharmaceutical composition particles) of Example 4.

### [Dissolution Test and Evaluation of Masking Properties of Unpalatable Taste]

A dissolution test and evaluation of masking properties of an unpalatable taste were performed as in Test Example 1. The results of the dissolution test are shown in Table 3. With respect to sertraline hydrochloride bulk powder and levocetirizine hydrochloride, all of the panelists felt the unpalatable taste strongly as indicated by "++" at 1 minute and thereafter.

**[Table 3]**

| | Paddle | Dissolution rate (%) | | | | | |
|---|---|---|---|---|---|---|---|
| | rotation rate (rpm) | 2 minutes | 5 minutes | 15 minutes | 30 minutes | 45 minutes | 60 minutes |
| Example 3 | 50 | 2.5 | 8.2 | 55.2 | 81.5 | 91.2 | 93.5 |
| Example 4 | 100 | 17.8 | 52.1 | 79.5 | 84.5 | 89.6 | 93.3 |

Evaluation of masking properties of an unpalatable taste was performed with respect to samples of Example 3 and Example 4, and even at 2 minutes after the particles were taken, all of the panelists did not feel a problematic unpalatable taste, which means that a clear effect of suppression of the unpalatable taste was obtained. In addition, in the dissolution test, the dissolution rate reached 80% or more at about 15 to 30 minutes after the beginning of the dissolution test, which shows that the samples had such drug release properties that rapid development of sufficient drug efficacy could be expected. With respect to Example 4, as described in [Test Example 3] below, since obvious particle aggregation occurred in the dissolution test vessel at 50 rpm, the test was performed at 100 rpm.

### [Test Example 3]

In pharmaceutical composition particles according to the present invention, when cracks or the like are formed on an outer layer in a dissolution test, the gelling swelling substance is exposed at the surface of the particles, and aggregation of the particles occurs in the dissolution test vessel due to the viscosity of the gelling swelling substance. This tendency is developed when a highly viscous gelling swelling substance is used. Thus, when the paddle rotation rate in the dissolution test is low (50 rpm), the particles form wet aggregates having viscosity in the test solution, and true dissolution data cannot be obtained. Thus, for the purpose of loosening the aggregation and obtaining true dissolution data, comparative examples with a paddle rotation rate of 100 rpm were performed.

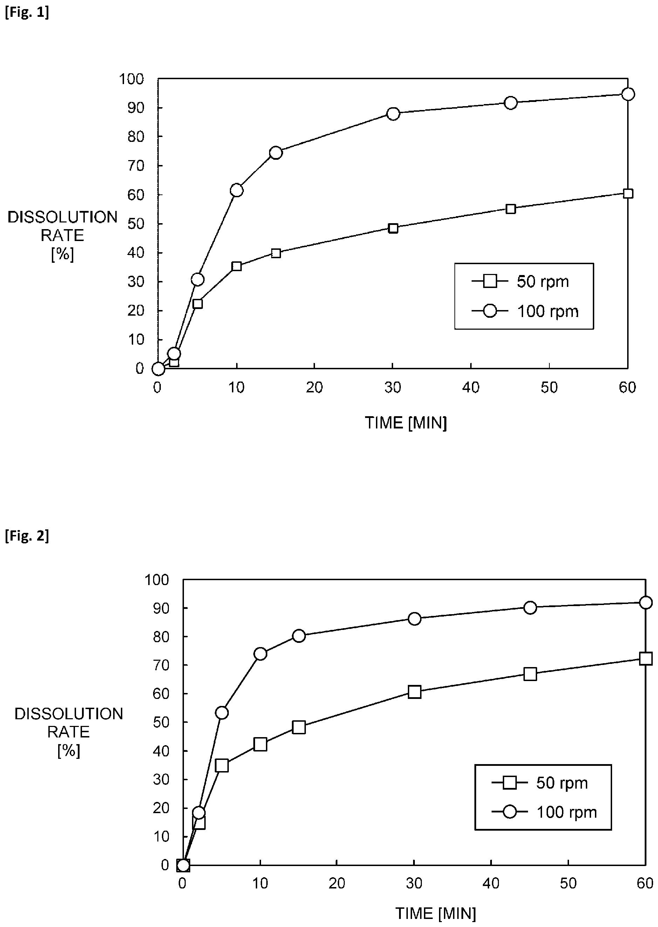

Using particles in Example 2 and Example 4, a dissolution test was performed according to the Japanese Pharmacopoeia Method 2 by means of an automatic 6-vessel dissolution test apparatus (manufactured by Toyama Sangyo Co., Ltd.). As a test solution, 900 mL of the 2nd fluid for dissolution test in the Japanese Pharmacopeia was used. The paddle rotation rates were 50 rpm and 100 rpm. The results of the dissolution test are shown in FIG. 1 and FIG. 2.

As shown in FIG. 1 and FIG. 2, in particles of Example 2 and Example 4, there is not much difference between 50 rpm and 100 rpm at 2 to 5 minutes. At 50 rpm, the dissolution rate fell extremely at 10 minutes and thereafter, and the dissolution rate did not reach 100% even at 60 minutes. It has been found that, from visual observation during the dissolution test, this was caused by aggregation of the particles formed at approximately 5 to 10 minutes, which clearly showed that the results did not reflect the drug release in vivo. At 100 rpm, the dissolution rate reached 80% or more at 15 to 30 minutes, which shows that aggregation was loosened and the true drug release was reflected.

Thus, when a gelling swelling substance having such a high viscosity that a viscosity of 2% solution at 25°C exceeds 50 mPa·s was used, and when strong aggregation of the particles occurs at a paddle rotation rate of 50 rpm, for the purpose of loosening the aggregation, it is meaningful to perform the test at 100 rpm.

### [Test Example 4]

With respect to pharmaceutical composition particles according to the present invention, drug release properties in solution having different pH were examined.

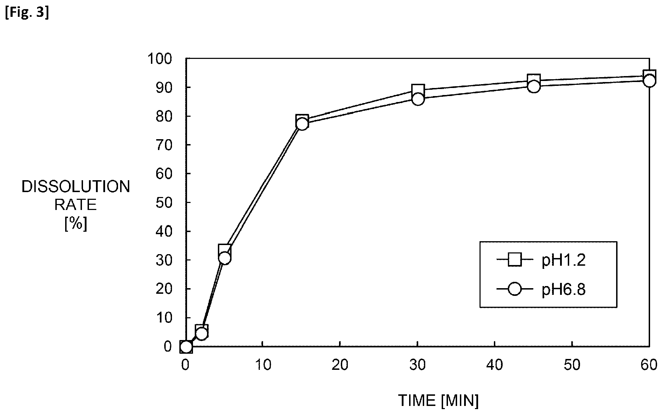

With respect to particles in Example 2, a dissolution test was performed according to the Japanese Pharmacopoeia Method 2 by means of an automatic 6-vessel dissolution test apparatus (manufactured by Toyama Sangyo Co., Ltd.). As test solutions, 900 mL of the 2nd fluid for dissolution test in the Japanese Pharmacopeia (pH 6.8) and 900 mL of the 1st fluid for disintegration test in the Japanese Pharmacopeia (pH 1.2) were used. To prevent aggregation of pharmaceutical composition particles in the dissolution test fluid, the tests were carried out at 100 rpm. The results are shown in Table 4 and FIG. 3.

**[Table 4]**

| | pH | Dissolution rate (%) | | | | | |
|---|---|---|---|---|---|---|---|
| | | 2 minutes | 5 minutes | 15 minutes | 30 minutes | 45 minutes | 60 minutes |
| Example 2 | 1.2 | 5.5 | 33.5 | 78.6 | 89.1 | 92.5 | 94.0 |
| | 6.8 | 4.5 | 30.8 | 77.5 | 86.1 | 90.5 | 92.5 |

There is no difference between the two test solutions, and it is found that the pharmaceutical composition particles according to the present invention are excellent preparations having similar drug release properties both in the stomach and in the intestinal tract.

### [Test Example 5]

Outer layer-coated particles were manufactured by using different water-insoluble substances for outer layers (Examples 5 and 6), and a dissolution test and evaluation of masking properties of an unpalatable taste were performed as in Test Example 1.

### [Examples 5 and 6]

In 760 g of ethanol, 36 g of AEA and 4 g of triethyl citrate were dissolved to prepare an AEA outer layer solution. In a mixed solution of 608 g of ethanol and 152 g of purified water, 40 g of AQOAT (AS-MG) was dissolved to prepare an AQOAT outer layer solution.

A tumbling fluidized bed coating granulating machine (model: MP-01) was charged with 200 g of drug/gelling agent layering particles (core particles) which were manufactured by the same way as in Example 1. The drug/gelling agent layering particles (core particles) were sprayed and coated with each of the outer layer solutions with agitation and fluidization, and dried to afford outer layer-coated particles.

With 800 g of the AEA outer layer solution, 200 g of the drug/gelling agent layering particles (core particles) were sprayed and coated, and dried to afford outer layer-coated particles, which were designated as outer layer-coated particles of Example 5. With 800 g of the AQOAT outer layer solution, 200 g of the drug/gelling agent layering particles were sprayed and coated, and dried to afford outer layer-coated particles, which were designated as outer layer-coated particles of Example 6.

Using the outer layer-coated particles of Example 5 and Example 6, a dissolution test and evaluation test of an unpalatable taste were performed as in Test Example 1. The results of the dissolution test are shown in Table 5.

**[Table 5]**

| | pH of test solution | Paddle rotation rate (rpm) | Dissolution rate (%) | | | | | |
|---|---|---|---|---|---|---|---|---|
| | | | 2 minutes | 5 minutes | 15 minutes | 30 minutes | 45 minutes | 60 minutes |
| Example 5 | 6.8 | 50 | 15.4 | 38.7 | 55.0 | 68.7 | 80.5 | 90.8 |
| Example 6 | 6.8 | 50 | 42.0 | 80.4 | 86.2 | 89.2 | 91.1 | 92.5 |

The results of the dissolution test with respect to the outer layer-coated particles of Example 5 show that dissolution rate in the test solution with pH 6.8 reached 90% or more at 60 minutes, which satisfied a criterion of rapid release. All of the panelists felt no bitter taste at 1 minute after the beginning of the test. Some panelists felt a bitter taste slightly after 2 minutes, but the bitter taste was not problematic. With respect to the outer layer-coated particles of Example 6, the particles were coated with an enteric coating agent which is dissolved in the 2nd fluid for the dissolution test (pH 6.8), and the particles had dissolution properties in which dissolution rate reached 80% or more at 5 minutes. However, since AQOAT was a film agent which did not dissolved in the oral cavity, all of the panelists did not feel a problematic unpalatable taste after 2 minutes. In addition, just to be sure, dissolution properties of the particles of Example 6 in water was measured (paddle rotation rate: 50 rpm), the particles had dissolution properties in which the dissolution rate reached 17.0% at 2 minutes, and 80.5% at 30 minutes.

### [Test Example 6]

Particles were manufactured by using different amounts of water-insoluble polymers contained in the outer layers (Example 7 to Example 9), and a dissolution test and evaluation of masking properties of an unpalatable taste were performed as in Test Example 1.

### [Example 7]

In a mixed solution of 291.6 g of ethanol and 32.4 g of purified water, 25.2 g of ETHOCEL 7 and 10.8 g of TC-5E were dissolved to prepare an outer layer solution. A tumbling fluidized bed coating granulating machine (model: MP-01) was charged with 180 g of drug/gelling agent layering particles (core particles) which were manufactured by the same way as in Example 2. The drug/gelling agent layering particles (core particles) were sprayed and coated with 360 g of the outer layer solution with agitation and fluidization, and dried to afford outer layer-coated particles of Example 7.

Using the outer layer-coated particles of Example 7, which were prepared so that the water-insoluble polymer contained in the outer layer accounted for 70% by weight of the total weight of the outer layer, a dissolution test was performed as in Test Example 1. Since particle aggregation in the dissolution test vessel was observed, the test was performed at a paddle rotation rate of 100 rpm. The results are shown in Table 6 together with the results of Example 2 (Table 3) in which the water-insoluble polymer (ETHOCEL 7) contained in the outer layer is the same and the water-soluble polymer contained in the outer layer accounted for 90% by weight of the total weight of the outer layer.

**[Table 6]**

| | Amount of water-insoluble polymer in outer layer (% by weight) | Paddle rotation rate (rpm) | Dissolution rate (%) | | | | | |
|---|---|---|---|---|---|---|---|---|
| | | | 2 minutes | 5 minutes | 15 minutes | 30 minutes | 45 minutes | 60 minutes |
| Example 2 | 90 | 100 | 4.9 | 33.5 | 80.1 | 87.1 | 91.5 | 93.9 |
| Example 7 | 70 | 100 | 12.8 | 40.5 | 83.2 | 89.1 | 92.4 | 95.9 |

According to the results of the dissolution test, it was found that the particles are rapid-release particles showing a dissolution rate of 80% or more at 15 minutes. On the other hand, with respect to the evaluation of an unpalatable taste, all of the panelists did not feel a problematic unpalatable taste at 2 minutes.

### [Example 8 and Example 9]

In a mixed solution of 291.6 g of ethanol and 32.4 g of purified water, 19.8 g of ETHOCEL 7 and 16.2 g of TC-5E were dissolved to prepare an outer layer solution. A tumbling fluidized bed coating granulating machine (model: MP-01) was charged with 180 g of drug/gelling agent layering particles (core particles) which were manufactured by the same way as in Example 2. The drug/gelling agent layering particles (core particles) were sprayed and coated with 360 g of the outer layer solution with agitation and fluidization, and dried to afford outer layer-coated particles of Example 8.

In a mixed solution of 291.6 g of ethanol and 32.4 g of purified water, 23.4 g of ETHOCEL 7 and 12.6 g of TC-5E was dissolved to prepare an outer layer solution. A tumbling fluidized bed coating granulating machine (model: MP-01) was charged with 180 g of drug/gelling agent layering particles (core particles) which were prepared by the same way as in Example 2. The drug/gelling agent layering particles (core particles) were sprayed and coated with 360 g of the outer layer solution with agitation and fluidization, and dried to afford outer layer-coated particles of Example 9.

Using the outer layer-coated particles of Example 8, which were prepared so that the water-insoluble polymer contained in the outer layer accounted for 55% by weight of the total weight of the outer layer, and using the outer layer-coated particles of Example 9, which were prepared so that the water-insoluble polymer contained in the outer layer accounted for 65% by weight of the total weight of the outer layer, an evaluation test of an unpalatable taste in the oral cavity was performed. As a result, both in the outer layer-coated particles of Example 8 and Example 9, a problematic unpalatable taste was not felt. However, with respect to the outer layer-coated particles of Example 8, it was found that, due to the gelling swelling substance exuded in the oral cavity, a phenomenon in which the fine particle strongly adhered to the mouth took place. Thus, it was found that the content of the water-insoluble substance included in the outer layer is preferably 60% by weight or more.

### [Test Example 7]

Using a spray-drying fluidized bed granulating apparatus, pharmaceutical composition particles were produced by a method for directly manufacturing substantially sphere-shaped drug-containing particles from a solution containing a binder in which a gelling swelling substance and a drug were dissolved or suspended. As in Test Example 1, a dissolution test and evaluation of masking properties of an unpalatable taste were performed.

### [Example 10]

In a mixed solution of 153.2 g of purified water and 612.7 g of ethanol, 43.2 g of HPC-L was dissolved to form a solution. In the solution, 72 g of ambroxol hydrochloride (average particle diameter: about 3 µm) and 100.8 g of CELLOGEN F-SC (finely pulverized material) were suspended, and the resulting suspension was used as a liquid raw material. Using the liquid raw material, in a splay drying fluidized bed granulator (model: MP-01-SPC, without inner tube), continuous spraying/layering was performed by means of a nozzle having a diameter of 1.2 mm under conditions of charge air temperature of 50 to 60°C and charge airflow rate of 0.65 to 0.7 m³/min, the resulting materials were dried, and then particles having a diameter of 105 µm or less were removed to afford core fine particles. In a mixed solution of 182.25 g of ethanol and 20.25 g of purified water, 20.25 g of ETHOCEL 7 and 2.25 g of TC-5E were dissolved to prepare an outer layer solution. A tumbling fluidized bed coating granulating machine (model: MP-01) was charged with 150 g of the core fine particles. The core fine particles were sprayed with 225 g of the outer layer solution with tumbling and fluidizing to afford outer layer-coated particles of Example 10.

Using the outer layer-coated particles of Example 10, a dissolution test was performed as in Test Example 1. With respect to this outer layer-coated particles manufactured by using CELLOGEN F-SC, since obvious particle aggregation in the dissolution test vessel was observed during the dissolution test at a paddle rotation rate of 50 rpm, the test was performed at a paddle rotation rate of 100 rpm. The results are shown in Table 7.

**[Table 7]**

| | Dissolution rate (%) | | | | | |
|---|---|---|---|---|---|---|
| | 2 minutes | 5 minutes | 15 minutes | 30 minutes | 45 minutes | 60 minutes |
| Example 10 | 3.4 | 15.5 | 56.5 | 80.5 | 90.1 | 93.1 |

According to the results of the dissolution test, it was found that the dissolution rate reached 80% or more at 30 minutes, and the particles satisfied a criterion of rapid release. On the other hand, all of the four panelists did not feel a problematic unpalatable taste at 2 minutes. Thus, it was found that a favorable result was also obtained by a method for directly manufacturing core particles from a gelling swelling substance and a drug, and coating the outer layer of each of the core particle with a water-insoluble substance.

### [Test Example 8]

Using the outer layer-coated particles of Example 1, an orally disintegrating tablet was prepared.

### [Example 11]

To 240 g of the outer layer-coated particles manufactured in Example 1, 240 g of additional granules, which were manufactured according to the formulation as described below, were added and mixed. Then, using magnesium stearate as an external lubricant, the mixture was compressed into tablets each having a weight of 480 mg at a tableting pressure of 7 kN by means of a punch having a diameter of 10.0 mm (R = 11.5 mm). The additional granules were manufactured as follows. A high speed mixing/granulating apparatus (VG-10, manufactured by Powrex Corporation) was charged with 1.06 kg of D-mannitol, 300 g of crystalline cellulose, 125 g of crospovidone, and 15 g of a sweetener. The contents were mixed, suitable amount of water was added, kneaded by a standard rotation speed of the impeller and the chopper for 5 minutes, and dried by means of a fluidized bed dryer (MP-01, manufactured by Powrex Corporation) at a charge air temperature of 80°C. Then, the resulting dried granules were subjected to particle size regulation using a 50-mesh sieve to obtain the above-mentioned additional granules. The resulting tablet disintegrated within 30 seconds after taken into the oral cavity, and the tablet did not give a problematic unpalatable taste for 2 minutes.

As described above, a pharmaceutical composition particle according to the present invention includes a core particle containing a water-soluble gelling swelling substance and a drug; and an outer layer which contains a water-insoluble substance and constitutes a coating provided on the outer side of the core particle. The gelling swelling substance preferably has a viscosity of 10 mPa·s or more in a 2% aqueous solution at 25°C. The outer layer preferably contains the water-insoluble substance in an amount of 60% by weight or more with respect to the total weight of the outer layer. The gelling swelling substance preferably has a swelling power (S) of 650 or more. The amount of coating of the outer layer is preferably 5% by weight or more and 50% by weight or less with respect to the core particle.

An orally disintegrating tablet according to the present invention contains any of the above-described pharmaceutical composition particles.

A method for manufacturing the pharmaceutical composition particle according to the present invention includes: a pulverization step of pulverizing a water-soluble gelling swelling substance so that the pulverized water-soluble gelling swelling substance has an average particle diameter of 15 µm or less; a core particle manufacturing step of mixing the gelling swelling substance pulverized in the pulverization step and a drug to manufacture a core particle; and an outer layer formation step of coating the core particle with a water-insoluble substance on the outer side of the core particle to form an outer layer. The core particle manufacturing step preferably includes a step of suspending the gelling swelling substance pulverized in the pulverization step and the drug in an organic solvent, and the organic solvent is preferably ethanol.

The embodiments and examples disclosed herein are for illustrative purpose only and should not be construed as limiting in any way. The scope of the invention is determined by the appended claims, but is not determined by the foregoing descriptions. All modifications within the scope of the claims and their equivalents fall within the scope of the invention.

## Claims

1. A pharmaceutical composition particle comprising:
a core particle containing a drug and a water-soluble gelling swelling substance; and
an outer layer which contains a water-insoluble substance and constitutes a coating provided on the outer side of the core particle.

2. The pharmaceutical composition particle according to claim 1, wherein the water-soluble gelling swelling substance has a viscosity of 10 mPa·s or more in a 2% aqueous solution at 25°C.

3. The pharmaceutical composition particle according to claim 1 or 2, wherein the outer layer contains the water-insoluble substance in an amount of 60% by weight or more with respect to the total weight of the outer layer.

4. The pharmaceutical composition particle according to any one of claims 1 to 3, wherein the water-soluble gelling swelling substance has a swelling power (S) of 650 or more.

5. The pharmaceutical composition particle according to any one of claims 1 to 4, wherein an amount of the coating of the outer layer is 5% by weight or more and 50% by weight or less with respect to the core particle.

6. An orally disintegrating tablet comprising the pharmaceutical composition particle according to any one of claims 1 to 5.

7. A method for manufacturing a pharmaceutical composition particle, comprising:
a pulverization step of pulverizing a water-soluble gelling swelling substance so that the pulverized water-soluble gelling swelling substance has an average particle diameter of 15 µm or less;
a core particle manufacturing step of mixing the pulverized water-soluble gelling swelling substance and a drug to manufacture a core particle; and
an outer layer formation step of coating the core particle with a water-insoluble substance on the outer side of the core particle to form an outer layer.

8. The method for manufacturing a pharmaceutical composition particle according to claim 7, wherein the core particle manufacturing step includes a step of suspending the water-soluble gelling swelling substance pulverized in the pulverization step and the drug in an organic solvent, and the organic solvent is ethanol.
